(19) Europäisches Patentamt
European Patent Office
Office européen des brevets

(11) **EP 2 713 874 B1**

(12) **EUROPEAN PATENT SPECIFICATION**

(45) Date of publication and mention
of the grant of the patent:
**29.04.2015 Bulletin 2015/18**

(51) Int Cl.:
***A61B 5/11*** *(2006.01)*

(21) Application number: **12792991.7**

(22) Date of filing: **25.05.2012**

(86) International application number:
**PCT/GB2012/051181**

(87) International publication number:
**WO 2012/164262 (06.12.2012 Gazette 2012/49)**

(54) **GAIT ASYMMETRY MEASUREMENT**

GANGASYMMETRIEMESSUNG

MESURE D'ASYMÉTRIE DE LA MARCHE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2011 GB 201108952**

(43) Date of publication of application:
**09.04.2014 Bulletin 2014/15**

(73) Proprietor: **OXFORD BROOKES UNIVERSITY Oxfordshire OX3 0BP (GB)**

(72) Inventors:
• **ESSER, Patrick**
**NL-6369 SV Simpelveld (NL)**

• **HOWELLS, Ken**
**Oxford**
**Oxfordshire OX3 0BP (GB)**
• **DAWES, Helen**
**Oxford**
**Oxfordshire OX3 0BP (GB)**
• **COLLET, Johnny**
**Oxford**
**Oxfordshire OX3 0BP (GB)**

(74) Representative: **Wilson, Alan Stuart**
**Barker Brettell LLP**
**Medina Chambers**
**Town Quay**
**Southampton SO14 2AQ (GB)**

(56) References cited:
**WO-A1-2010/073044      US-A1- 2005 004 495**

**Description**

**Field of the Invention**

The present invention relates to the measurement of gait and in particular to the measurement of asymmetry in gait.

**Background to the Invention**

Variability is always present in human movement. Commonly variability is seen as noise, thus is unwanted and removed by data processing. Variability analysis has been used to indentify human gait patterns, but also for clinical purposes such as analysing pathological gait. Studies have found increased step time variability in elderly subjects compared to young adults. Increase in stride-to-stride variability within elderly subjects has also been found.
Parkinson's disease (PD), a progressive disorder of the central nervous system, presents with resting tremor, short slow steps, decreased centre of mass (CoM) movement and an increase in variability of temporospatial parameters of gait such as stride length and step time. Research has also shown that people with idiopathic Parkinson's disease have a higher gait asymmetry compared to age matched controls. Studies have also been carried out which suggest that gait asymmetry is common when looking at temporospatial parameters in both typically developed adults (TDA) and PD. In these studies fractal analysis has been used, which relies on longer walks (i.e. 2 and 6 minute walking tests). An issue with these studies is that the data sets required are relatively large. Gathering this amount of data can be seen as time consuming and therefore rather stressful for the participant, especially those with clinical conditions.
As described, for example, in WO2010/073044 inertial measurement unit (IMU) technology can be used to measure centre of mass (CoM) movement, providing a quick and relatively cheap way of gathering larger amounts of data over relative few steps where a high sample frequency is used.

**Summary of the Invention**

The present invention provides a system for measuring variation in the gait of a subject, the system comprising measuring means arranged to measure variations in the position of the subject while the subject takes a series of steps. The position may be a vertical position, but may be position in any direction, or along any axis. The direction or axis may be close to vertical, or may be a horizontal direction or axis. The system may further comprise processing means. The system may further comprise display means. The processing means may be arranged to identify a plurality of points in a first one of the steps and a plurality of points in a second one of the steps. The processing means may be arranged to identify a plurality of pairs of the points. Each pair may comprise one point in each of the steps. The processing means may be arranged to determine a value of height for each of the points in each of the pairs. The processing means may be arranged to control the display means to produce a display plotting the heights of the two points in each pair against each other.
The processing means may be arranged to identify the first and second steps as consecutive steps in the series. However, the first and second steps may be selected from anywhere in the series. Where the system is arranged to measure asymmetry between the legs of a subject, the first and second steps may be selected to be on different legs. However in some cases they may be different steps on the same leg where variation over time between steps on the same leg is being measured.
The processing means may be arranged to identify each pair of points so that the time interval between the two points in each pair is the same. Alternatively the time interval may be different for different pairs of points. There may be some other relationship between the pairs of points, such as their relative position within the step cycle.
The processing means may be arranged to identify a series of points through the series of steps and to include each of the series of points in one of the pairs. This allows the use of a constant (or regularly varying) sampling interval throughout the test walking period.
The processing means may be arranged to define a coordinate system having two axes representing the heights of the two points in a pair. This can allow the heights of each pair of points to be represented by a position within the coordinate system. The processing means may be arranged to identify the positions in the coordinate system for each of the pairs of points. The processing means may be arranged to control the display so as to indicate those positions, for example by means of respective markers. The markers may comprise dots or crosses or be of any other suitable shape.
The processing means may be arranged to analyse the positions associated with the pairs of points and to calculate a parameter of the positions. The parameter may be a statistical parameter, for example a standard deviation of distance from a point or a line, or it may be an average position.
The processing means may be arranged to calculate the position of a line having a predetermined relationship to the positions associated with the pairs of the points. The line may be a straight line, or it may be a curve. For example it may be an oval, or a circle. The processing means may be arranged to control the display to display the line.

The present invention further provides a method of measuring variation in the gait of a subject. The method includes measuring variations in vertical position of the subject while the subject takes a series of steps. The method may comprise any one or more of: identifying a plurality of points in a first one of the steps and a plurality of points in a second one of the steps; identifying a plurality of pairs of the points, each pair comprising one point in each of the steps; determining a value of height for each of the points in each of the pairs; and, producing a display plotting the heights of the two points in each pair against each other.

The first and second steps may be identified as consecutive steps in the series. The pairs of points may be identified so that the time interval between the two points in each pair is the same.

The method may include identifying a series of points through the series of steps and including each of the series of points in one of the pairs.

The method may include defining a coordinate system having two axes representing the heights of the two points in a pair, so that the heights of each pair of points can be represented by a position within the coordinate system.

The method may include identifying the positions in the coordinate system for each of the pairs of points, and controlling the display so as to indicate those positions.

The method may include analysing the positions associated with the pairs of points and calculating a parameter of the positions.

The method may include calculating the position of a line having a predetermined relationship to the positions of the points, and controlling the display to display the line.

The present invention further provides a system for measuring variation in the gait of a subject, the system comprising a memory arranged to store data recording variations in vertical position of the subject while the subject takes a series of steps, processing means, and display means. The processing means may be arranged to identify a plurality of points in a first one of the steps and a plurality of points in a second one of the steps. The processing means may be arranged to identify a plurality of pairs of the points, each pair comprising one point in each of the steps. The processing means may be arranged to determine a value of height for each of the points in each of the pairs. The processing means may be arranged to control the display means to produce a display plotting the heights of the two points in each pair against each other.

Preferred embodiments of the present invention will now be described by way of example only with reference to the accompanying drawings.

**Brief Description of the Drawings**

Figure 1 is a diagram of a subject with an inertial measurement unit used in an embodiment of the invention;

Figure 2 is a diagram of a computer system according to an embodiment of the invention arranged to analyse data from the IMU of Figure 1;

Figure 3 is a computer simulated plot of height of the CoM of the subject of Figure 1 as a function of time with variation in step length;

Figure 4 shows the plot of Figure 3 superimposed on a copy of the same plot shifted in time by the duration of one step;

Figure 5 is a plot of CoM excursion from the shifted plot as a function of the CoM excursion from the original plot, with values for each step on the horizontal axis plotted against values for the previous step on the vertical axis;

Figure 6 is a plot of measured height of the CoM of the subject of Figure 1 during walking as a function of time;

Figure 7 is a plot similar to Figure 5 for the data of Figure 6;

Figure 8 is a diagram showing parameters of the plot of Figure 7 which can be quantified in an embodiment of the invention;

Figures 9a - 9h are computer simulated plots similar to that of Figure 5 for sine waves of equal amplitude and wavelength, but out of phase by $180^0$ (a), $225^0$ (b), $270^0$ (c), $315^0$ (d), $360^0$ (e), $45^0$ (f), 90° (g) and $135^0$ (h);

Figures 10a-d are computer simulated plots similar to that of Figure 5 for two sine waves (frequency 10.1Hz, amplitude 5 and 7cm) being out of phase by $180^0$ (a), $170^0$ (b) $360^0$ (c) and $90^0$ (d);

**Figures 11a and 11b** are computer simulated plots based on three sine waves (constant amplitude of 5cm and phase shift of 180°) representing a change in step length (a) and step frequency (b); and

**Figure 12** is a set of three plots similar to that of Figure 5 for PD sufferers and three plots for typically developed adults generated from measured data.

**Description of the Preferred Embodiments**

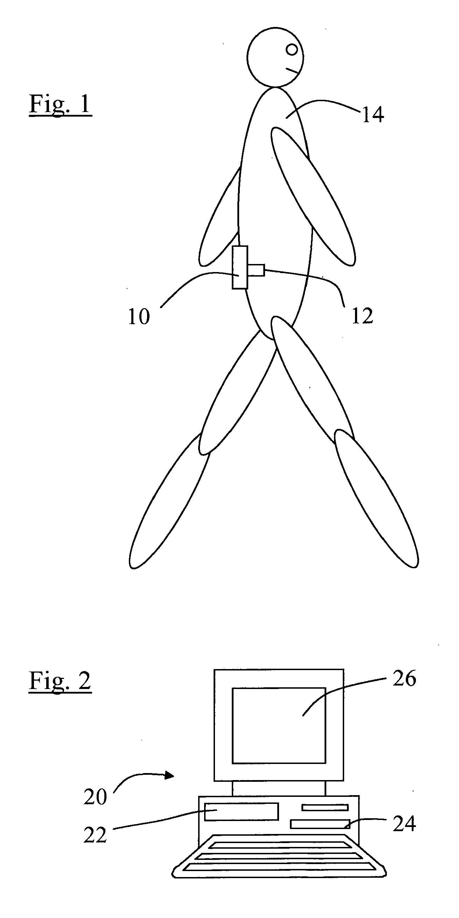

Referring to Figure 1 an inertial measurement unit (IMU) 10 is attached to a subject 14 by means of adhesive tape 12 . The IMU is of a known form and comprises a number of accelerometers and is arranged to identify the vertical direction using the accelerometers and to measure acceleration in the vertical direction. It is then arranged to calculate vertical velocity and hence vertical position continuously and to store the calculated vertical position at a series of sample points in time. The vertical position can be relative to any reference position or height, which in this case is the position of the CoM at the start of measurement when the subject is standing still. In this embodiment the measured vertical position is measured and defined by the IMU 10 as a CoM excursion, which is the difference between the actual vertical position of the CoM of the subject at a point in time and the reference, starting vertical position of the CoM.

Referring to Figure 2 a computer system 20 is arranged to receive the CoM excursion data collected over a test period and analyse it and use it to generate various displays which are arranged to help with the identification of various characteristics of the gait of the subject. The computer system comprises a processor 22, a memory 24 and a display screen 26. In conventional manner the memory is arranged to store the data collected by the IMU 10, and also to store a program which controls the processing of the data by the processor 22 and controls the display screen 26. It will be appreciated that the amount of processing of the raw sensor signals that is carried out in the IMU 10 and the amount that is done by the computer system 20 is arbitrary.

The CoM movement of the subject can be described using an inverted pendulum model of the gait of the subject. This model describes the mechanical energetic state during a gait cycle in which the CoM excursion plotted as a function of time behaves like a sine wave having a sequence of peaks and troughs, with each step producing one cycle of the sine wave from the bottom of one trough to the bottom of the next. It will be appreciated that any height measure will give the same basic shape, with the reference height relative to which the height is measured being to some extent arbitrary. In this embodiment of the invention further analysis using a non-linear method is performed on the CoM excursion data by the computer system 20, whereby CoM Excursion (CoM Excursion$_i$) at each point in one step is plotted against the CoM Excursion (CoM Excursion$_{i-1}$) for the same point in the previous step.

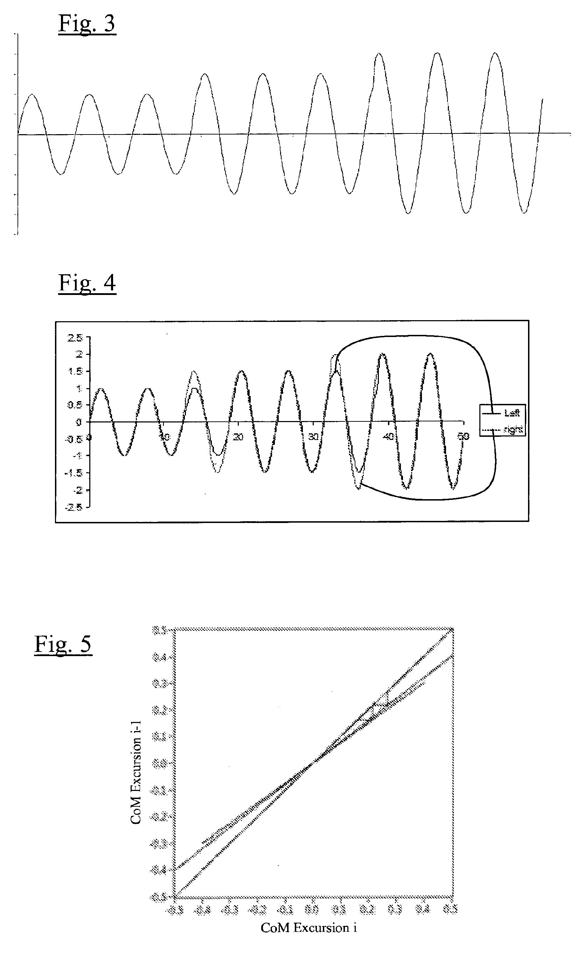

A computer generated example of a plot of CoM excursion as a function of time is shown in Figure 3. This shows simulated results for a series of nine walking steps with CoM excursion from the starting point shown on the vertical axis and time on the horizontal axis, with the step length increasing after the first three, and the next three steps. Sample frequency in the simulation is set as 100Hz which corresponds to the sample rate for the inertial measurement unit (IMU). This is a suitable sample frequency for gait measurement but in practice the sample rate of the IMU can vary, for example up to 120Hz, or higher if particularly high resolution is required. Therefore the time interval between sample height measurements is equal to 0.01seconds. The plot of Figure 3 is over a period of 5 seconds. Therefore there are 500 sample points on the plot, and as there are nine steps there are about 55 sample points per step.

Referring to Figure 4, in order to measure variation in gait, the computer system 22 is arranged to compare the CoM excursion for each sample point in each step with the CoM excursion at the corresponding point in the previous step. It will be appreciated that this compares the CoM excursion for a step on one leg with a corresponding CoM excursion for a step on the other leg. In order to do this, the plot of Figure 3 is copied and shifted by one step length, resulting in two CoM excursion plots superimposed on each other as shown. Each point on one of the curves is therefore aligned with the corresponding sample point in the previous step in the other curve. Where a step is the same as the previous step, the curves are aligned, and where there is a difference between one step and the next this can be seen as a divergence between the two curves.

Referring to Figure 5, the computer is then arranged to generate a two dimensional plot (referred to herein as a non-linear plot) of the CoM excursion for each point on one axis (the horizontal axis) against the CoM excursion for the corresponding point one step previously on a perpendicular axis (the vertical axis). For all cases where the two excursions are equal, the plot will include a point on the line at 45° through the point (0,0). The points in the third peak and third trough where the maximum excursion of the current step is 1.5cm and of the previous step is 1.0cm will generate a line passing through (0,0) and the point (1.5,1). The points in the sixth peak and sixth trough where the maximum excursion of the current step is 2cm and of the previous step is 1.5cm will generate a line passing through (0,0) and the point (2,1.5). It will be appreciated that in this example as the two superimposed plots are in phase, because the steps are of a constant frequency, the lines on the plot of Figure 5 all pass though the point (0,0). However if the steps vary in period, then the original and shifted curves will not cross the horizontal axis at the same time, so a plot similar to Figure 5 will not pass through that point.

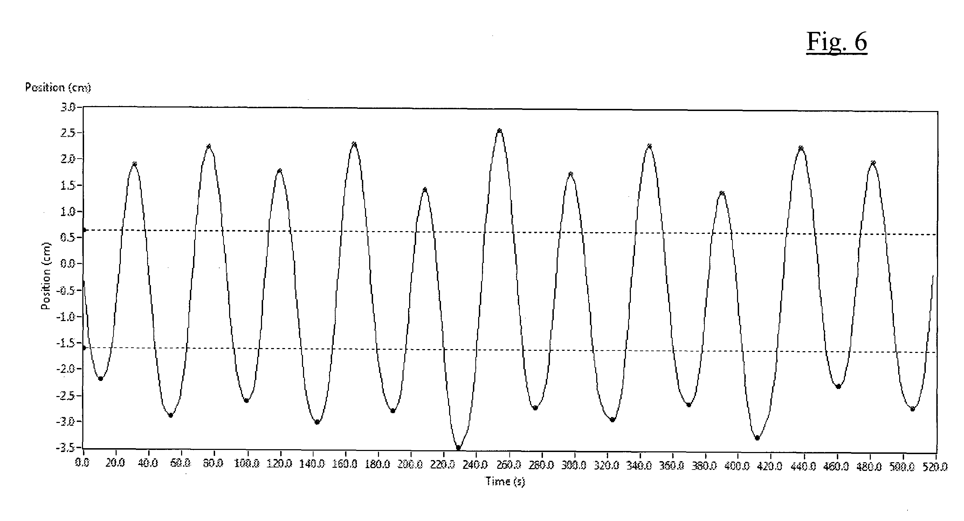

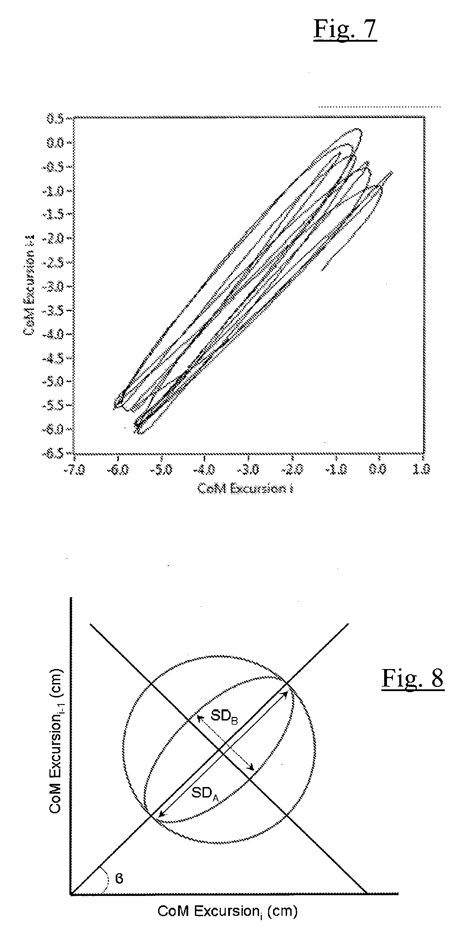

This is illustrated further with reference to Figure 6 which shows a plot of actual measured CoM excursion as a function of sample number, with a sampling frequency of 100Hz, for a real subject walking for 10m. Superficially it can be seen that the oscillations in CoM excursion alternate between higher and lower amplitudes, suggesting a difference between the steps of the left and right legs, though there is obviously significant variation in the steps for each leg. Plotting the CoM excursion (CoM Excursion i) for each sample point against the CoM excursion (CoM Excursion i-1) for the sample point a fixed time (approximately equal to one step length) earlier results in a plot shown in Figure 7. This plot shows a line joining each of the points that is produced for a respective sample time in the CoM excursion plot. The shape of the cluster of points, or the line joining them as in the plot of Figure 7, can be analysed to determine various parameters of the plot which in turn can be used as a measure of various characteristics of the gait of the subject.

It will be appreciated that, for the shape of the cluster to be analysed in a useful way, there need to be sufficient points in the plot, and therefore sufficient sample points of measured height. In the plot of Figure 6 there are approximately 40 sample points per step. Fewer sample points could be used, but an average of at least 10 per step is needed over the test period to be able to gather sufficient data over a relatively short test time. Also while it is simplest and desirable for the sample points to be equally spaced in time, there can be some variation in the time interval between sample points, for example to have more sample points around specific parts of the step cycle. In some cases each pair of points may be selected on the basis of their position within the step cycle. For example if each step is considered as a sine wave and the time within each step is defined as a fraction of the whole of that step, such as in terms of an angle between 0 and 360°, then the pairs of points could be selected as those having the same relative (angular) position with in the step cycle.

In order to better understand this analysis the theoretical exploration of these CoM plots by means of generated sine waves was performed in LabVIEW8.5 by means of a sine-generator which varied frequency, amplitude and phase shift. In order to mimic changes typically observed over a 10 metre walk the following components were altered:

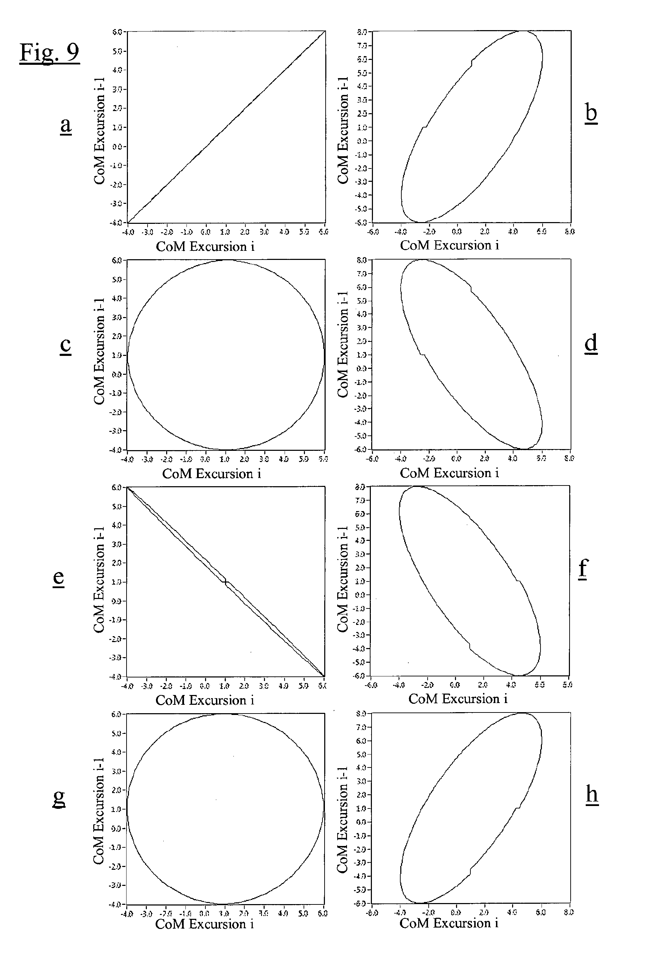

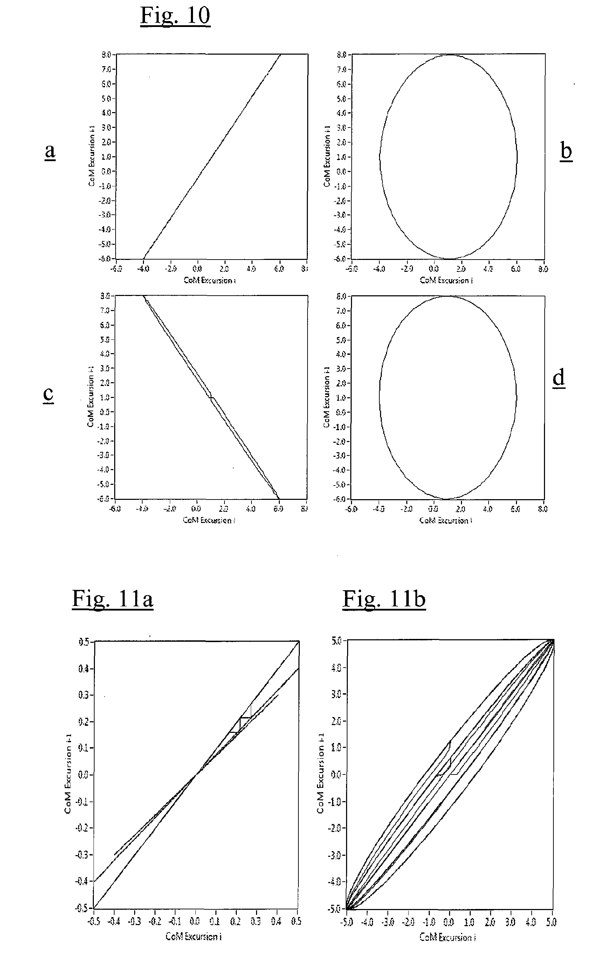

1) Phase shift was generated with 45 degree steps in order to explore potential phase shifts of the vertical CoM during human walking (Figure 9)
2) Amplitude ranges from 5 to 7cm, which represent typical vertical CoM movement during human walking (Figure 10)
3) Frequency values vary between 10-10.4Hz, representing the change in walking frequency during a 10 metre walk (Figure 11).

For each case a non-linear plot similar to that of Figures 5 and 7 was produced and analysed.

The analysis used for these plots will now be described with reference to Figure 8, and it will be appreciated that the computer 20 is arranged to perform this analysis on each set of data obtained from measurement of a subject over a short walking distance, and that the parameters identified can be used by the computer system 20 to analyse the measurement data from the subject.

A least square best fit straight line is plotted against the cloud of points in the CoM excursion plot and the line is plotted as a function $f = ax + b$, where $a$ is the gradient of the line and $b$ is the intercept.

The gradient is converted to an angle $\beta$ (degrees) measured from the horizontal axis in the anticlockwise direction towards the vertical axis as shown in equation (1).

$$\beta = \tan^{-1}(a) \cdot (180/\pi) \qquad (1)$$

A computer generated sine wave which assumes consistency and therefore no variability, would result in a value of $\beta = 45^0$.

A circle is fitted onto the data in order to find the origins of the cloud ($x_0, y_0,$) with the radius ($SD_A$) of the given data cloud as shown in equation (2)

$$SD_A = \sum_{i=1}^{n} ((x_i - x_0)^2 + (y_i - y_0)^2 - r^2)^2 \qquad (2)$$

which leads to a linear equation in $x_0, y_0,$ where ($x_i, y_i$) are the given points, ($x_0, y_0$) is the origins or midpoint and r is the unknown radius. Using the previously fitted sum of least squares the data is de-trended by subtracting the outcome of $y_i = ax_i + b$ from CoM Excursion$_{i-1}$ after which the standard deviation around the best fit straight line is calculated ($SD_B$).

An ellipse is fitted around the spread of data based on two standard deviations one $SD_A$ of position measured in the direction parallel to the best fit straight line, which is the length of the ellipse, and the other $SD_B$ of position measured in the direction perpendicular to the best fit straight line, which is the width of the ellipse. Ratio $\forall$ derived between $SD_A$ and $SD_B$ is determined to describe the ellipse. Furthermore angle $\beta$ shows the direction of the best fit straight line through the data points indicating a level of symmetry as shown in Figure 8.

Referring to Figure 9, two sine waves (being theoretically non variable) generated within LabVIEW8.5 containing a frequency of 10.1Hz, amplitude of 5cm with an intersect of 1cm, with zero phase shift between them, sampled at 100Hz will result in an angle $\beta$ - $45^0$ with an $R^2$ value of 1.00, $SD_A$ of 4.98cm and $SD_B$ of 0. When changing the phase shift (in steps of 45 degrees) of $CoM_{i-1}$ the plots will change according to the plots shown in Figures 9a to 9h. Specifically Figure 9 shows non-linear plots based on equal sine waves (frequency 10.1Hz, amplitude 5cm) while being out of phase by $180^0$ (A), $225^0$ (B) $270^0$ (C), $315^0$ (D), $360^0$ (E), $45^0$ (F), $90^0$ (G) and $135^0$ (H). It becomes clear that the sine wave produced data clouds rotate around their own axes (anti-clockwise) with change in phase shift.

CoM vertical displacement can be variable with differing limb or stride length. In order to explore the theoretical models two sign waves were generated with an amplitude of 5cm and 7cm respectively which represents typical human walking. The remaining configurations were similar to the previously used sine wave. Figure 10 shows the generated plots where it becomes visible that any variance in the CoM excursion results in a change in $\beta$. Specifically Figure 10 shows non-linear plots based on two sine waves (frequency 10.1Hz, amplitude 5 and 7cm) with different while being out of phase by $180^0$ (A), $170^0$ (B) $360^0$ (C) and $90^0$ (D).

Furthermore changes such as walking speed can be related to an increase in step length and cadence. Change in step length will result in a change of CoM vertical excursion when assuming the inverted pendulum model. The effects of the variability of step length is shown by creating three sine waves with different amplitudes (3, 5 and 7cm respectively) representing typical vertical CoM excursions during human walking which effect is visible in Figure 11 a. Analysis with three sine waves with different frequency (10Hz, 10.1Hz and 10.2Hz), in which the change (steps of 0.1Hz) represents human gait step time or cadence variability is shown in Figure 11b.

In use, to measure the variability of gait of a subject, the IMU 10 is strapped to the subject, and the subject walks a short distance, for example around 10 meters. During this time the IMU calculates and stores the height data for each of the sample points. The data is then input from the IMU to the computer 20 which is arranged to select pairs of sample points as described above and to generate a data set associating the two heights for each pair of sample points, for example as simple height values, or as coordinates on a plot such as the non-linear plot of Figure 5. The computer is then arranged to control the display screen 26 to display the non-linear plot of data points, each point having a position defined by the two height values of its respective pair of sample points. The computer is also arranged to calculate the best fit straight line, circle, and oval, as described above with reference to Figure 8, and to display those superimposed on the plot of the data points as shown in Figure 11. The values of $\beta$, $SD_A$, $SD_B$ and $\forall$ are also calculated and displayed on the screen. The plot itself or the numerical values calculated, or both can then be used as measures of parameters of the gait of the subject.

From the above analysis it will be clear that the computer system can be arranged to analyse the data in various different ways whilst still providing a similar measurement of gait variability. For example instead of each pair or sample points which are plotted against each other being a fixed number of samples (and hence a fixed time) apart, the data can be divided into separate steps and the first point in the left leg step plotted against the first point in the right leg step, and subsequent pairs of point plotted against each other. Alternatively the data can be separated into left leg and right leg steps, and then the left leg step data combined to form a first sine wave and the right leg data combined to form a second sine wave, and then corresponding points in the two sine waves plotted against each other. In a further alternative, rather then comparing contralateral data, i.e. data from opposite legs, ipsilateral data comparison is made in which the data for one step of one leg is compared with data for a different step of the same leg. For example the data can be divided into left and right leg steps, each of which is combined together to form left and right leg plots each being an approximate sine wave over several steps. The left leg plot is then shifted so that each left leg step is compared with the previous left leg step, and the same is done for the right leg data.

Study

Participants

Data collected from participants suffering from Parkinson's disease were analysed, and data from aged matched typical developed adults were also analysed.

Procedure

A Parkinson's Disease Questionnaire (PDQ) was administered for people with PD before partaking in this study.

Participants walked over a ten-metre walkway free of obstacles at their self selected walking speed. Participants started at a static position at the zero-point and came to a complete stop at the ten-metre line. The duration of the walk was recorded by a stopwatch. An IMU was placed over the projected CoM located over the fourth lumbar vertebrae, measuring at a sample frequency of 100Hz.

Analysis

IMU data was analysed by a program written in LabVIEW 8.5 (National Instruments, Ireland) to obtain vertical position. Temporal and spatial gait parameters were calculated according to Zijlstra's inverted pendulum model resulting in stride length and walking speed ($v_I$). $\beta$, $SD_A$, $SD_B$ and $\forall$ were derived by applying the non-linear method described above.

TDA and PD group were compared using an independent t-test on stride length and walking speed as well as $\beta$, $SD_A$ and $SD_B$. Furthermore a Pearson's regression test was used to test for a relationship between walking speed and $\beta$ for both PD and TDA. $\forall$ was tested by an independent t-test between PD and TDA.

Results

Descriptive measurements are displayed in Table 1.

Table 1 descriptive measurements showing mean for Barthel Index (BI) and Parkinson's disease questionnaire (PDQ) for typical developed adults (TDA) and people with Parkinson's disease (PD) where an asterisk shows a significant difference between groups (p<0.05). Sex is indicated as the number of males (M) taking part.

| Diagnosis | n Sex | PDQ scores | Age (years) | Diagnosis (years) | Stride Length (m) | Speed (ms$^{-1}$) | Cadence (steps/min) |
|---|---|---|---|---|---|---|---|
| TDA | 10 M = 6 | | 66.4 ± 4.4 | | 1.31 | ± 0.17 1.36 ± 0.33 | *121.2 ± 5.6 |
| PD | 14M=9 | 26 range 43-664.5 ± | 6.9 | 6.3 ± 3.9 | 1.29 ± 0.21 | 1.14 ± 0.24 | *111.5 ± 11.6 |

From the theoretical exploration of this non-linear analysis it became clear that $\beta$ is affected by a change in step length, $SD_A$ is affected by a change in step frequency as well as step length, $SD_B$ is affected by a change in step frequency and $\forall$ is the ratio between $SD_A$ and $SD_B$ defined as $SD_A/SD_B$.

An independent t-test showed no significant difference for stride length and cadence between TDA and PD participants (*p*=.615 and *p*=.342). However, a difference was found for walking speed (*p*=.041). Moreover an independent *t*-test between the TDA and PD group revealed a significant difference for $\beta$ (p=.010) and $SD_A$ (p=.004). No difference was found between groups for $SD_B$ (p=.385) and $\forall$ (*p*=.830). Results for each group can be found in Table 2.

Table 2 Outcomes from non-linear method applied to gait in typical developed adults (TDA) and Parkinson's disease (PD) showing the angle ($\beta$) of the least square fit with SDA and SDB describing the standard deviations of the non-linear plot with ratio $\forall$. An asterisk indicates a significant difference between both groups.

| Diagnosis | $\beta$ | $SD_A$ | $SD_B$ | $\forall$ |
|---|---|---|---|---|
| TDA | 42.0 ± 1.8 * | 1.9 ± 0.5 * | 0.4 ± 0.1 | 4.6 ± 3.9 |
| PD | 39.4 ± 3.9 * | 2.5 ± 0.5 * | 0.3 ± 0.1 | 7.8 ± 2.0 |

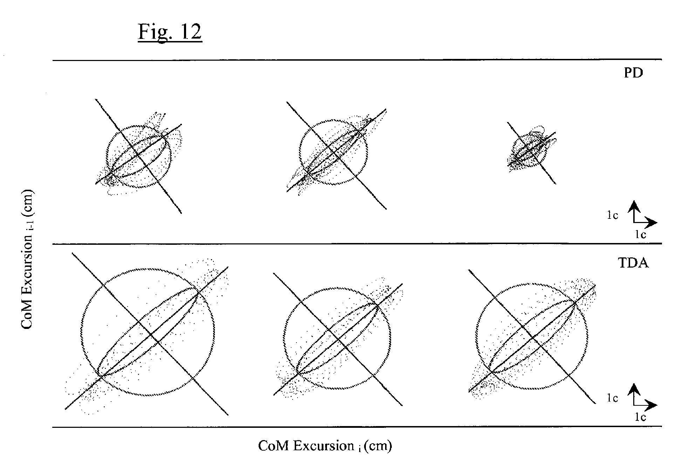

No correlation was found between $\beta$ and walking speed for PD (r2=.001 p=.996) or TDA (r2=.060 p=.810). Three representative analysis figures for each condition are shown in Figure 12.

Discussion

This study found that a non-linear analysis performed on CoM motion can be used to differentiate PD from TDA collected using IMUs over a 10 metre walk, whereas standard spatiotemporal parameters over the same distance could not. These findings are important as they promote the possibility of utilizing a non-linear variability analysis to objectively quantify gait variability and symmetry over a small sample frame, thus allowing people with PD at all stages of the disease to be monitored.

Previously, reduced step length has been reported as one of the key features of PD gait. Indeed, it has also been suggested that variability analysis may be used to closely monitor and describe gait disorders than measurements based on mean values of temporospatial walking parameters. The results of this study support this as, whilst there was no difference in step length, $\beta$ and $SD_A$ showed a significant difference between TDA and PD. Thus PD could be differentiated from TDA based on CoM variability (Figure 12). The significant difference in $SD_A$ may be due to increased step length variability reflected in CoM excursion. Although it should be noted that walking speed was significantly reduced for PD when compared to TDA, which has been reported previously.

Considering the novelty of this approach in exploring gait, a range of simulated CoM motions were modelled and run through the non-linear analysis in order to better understand the changes observed in PD. Group stride length variance observed in the data collected during this research, was 17cm for TDA and 21cm for PD during a 10 metre walk without step initiation. Assuming a constant leg length an increase in stride length of 17 and 21cm TDA and PD would increase the vertical CoM excursion by approximately 0.3 and 0.5cm. As shown in Figure 11a the effect of a simulated increase in step length of these values would change the angle of the non-linear plot by 3 and 5 degrees respectively. But it is worth noticing that this diversion in angle will only occur for one gait cycle when the change in step length occurred. Once the CoM excursion is symmetric and in phase between left and right after the asymmetrical cycle, $\beta$ will return to 45 degrees. Therefore it becomes visible that is influenced by a change in step length, $SD_B$ is affected by a change in step frequency, $SD_A$ is affected by both a change in step length as well as step frequency and $\forall$ is the ratio between the change in step frequency versus step length.

As seen in Figure 11b an increase in frequency will enlarge $SD_B$ as measured over the 10 metre walk. An increase of 0.2Hz walking frequency represents an increase of 12 steps per minute. As seen in Table 1 cadence variance is 5.6 for TDA and 11.6 for PD. A larger change in frequency results in a greater variance of $SD_B$. Perfect symmetry in lower limb stepping frequency causes the plots to assume a $SD_B$ equal to zero. Indeed, with variability in CoM excursions due to step length variability in combination with step frequency variability a phase shift is expected. As shown in Figure 9 phase shifts will change $\beta$, $SD_A$ and $SD_B$. During this study, however, no phase shift was detected within either TDA or PD measurements.

Novel methods have often been used to look into gait in more depth. For example fractal dynamic analysis has been used in TDA and PD to explore stride-to-stride fluctuations. An increase in stride-to-stride variability both in stride length as step time has been observed in early and late stages of PD. Our findings are in agreement with these previous findings by showing an increase in stride length variability ($SD_A$) as well as an increase in stride-to-stride symmetry ($\beta$) within a variety of PD. Despite a visual decrease in $SD_A$ and $SD_B$ in Figure 12, only $SD_A$ shows a significant difference between PD and TDA. This could be explained by the reduced stride length which has also been reported by PD research. Furthermore it is noticeable that the ratio $\forall$ does not show a significant difference between PD and TDA. This indicates that the symmetry variability $(SD_B)$ decreases by reducing stride length variability $(SD_A)$.

Thus embodiments of the present invention may provide a valuable measure for clinical use. Whilst the study described above was conducted for PD this method has the potential to indicate the severity of gait impairment in PD and other populations. The present invention can therefore provide a methodology that can assist in early diagnosis in people with PD and monitor their gait during deep brain stimulation. It may also be possible to use these gait parameters to more accurately monitor efficiency of medication in PD.

## Claims

1. A system for measuring variation in the gait of a subject, the system comprising measuring means arranged to measure variations in vertical position of the subject while the subject takes a series of steps, processing means, and display means, wherein the processing means is arranged to identify a plurality of points in a first one of the steps and a plurality of points in a second one of the steps, to identify a plurality of pairs of the points, each pair comprising one point in each of the steps, to determine a value of height for each of the points in each of the pairs, and to control the display means to produce a display plotting the heights of the two points in each pair against each other.

2. A system according to claim 1 wherein the processing means is arranged to identify the first and second steps as consecutive steps in the series.

3. A system according to claim 1 or claim 2 wherein the processing means is arranged to identify each pair of points so that the time interval between the two points in each pair is the same.

4. A system according to any foregoing claim wherein the processing means is arranged to identify a series of points through the series of steps and to include each of the series of points in one of the pairs.

5. A system according to any foregoing claim wherein the processing means is arranged to define a coordinate system having two axes representing the heights of the two points in a pair, so that the heights of each pair of points can be represented by a position within the coordinate system, and the processing means is arranged to identify the positions in the coordinate system for each of the pairs of points, and to control the display so as to indicate those positions.

6. A system according to claim 5 wherein the processing means is arranged to analyse the positions associated with the pairs of points and to calculate a parameter of the positions.

7. A system according to claim 5 or claim 6 wherein the processing means is arranged to calculate the position of a line having a predetermined relationship to the positions associated with the pairs of the points, and to control the display to display the line.

8. A method of measuring variation in the gait of a subject, the method including measuring variations in vertical position of the subject while the subject takes a series of steps, identifying a plurality of points in a first one of the steps and a plurality of points in a second one of the steps, identifying a plurality of pairs of the points, each pair comprising one point in each of the steps, determining a value of height for each of the points in each of the pairs, and producing a display plotting the heights of the two points in each pair against each other.

9. A method according to claim 8 wherein the first and second steps are identified as consecutive steps in the series.

10. A method according to claim 8 or claim 9 wherein the pairs of points are identified so that the time interval between the two points in each pair is the same.

11. A method according to any of claims 8 to 10 including identifying a series of points through the series of steps and including each of the series of points in one of the pairs.

12. A method according to any of claims 8 to 11 further comprising defining a coordinate system having two axes representing the heights of the two points in a pair, so that the heights of each pair of points can be represented by a position within the coordinate system, and identifying the positions in the coordinate system for each of the pairs of points, and controlling the display so as to indicate those positions.

13. A method according to claim 11 or claim 12 comprising analysing the positions associated with the pairs of points and calculating a parameter of the positions, or including calculating the position of a line having a predetermined relationship to the positions associated with the pairs of points, and controlling the display to display the line.

14. A system for measuring variation in the gait of a subject, the system comprising a memory arranged to store data recording variations in vertical position of the subject while the subject takes a series of steps, processing means, and display means, wherein the processing means is arranged to identify a plurality of points in a first one of the steps and a plurality of points in a second one of the steps, to identify a plurality of pairs of the points, each pair comprising one point in each of the steps, to determine a value of height for each of the points in each of the pairs, and to control the display means to produce a display plotting the heights of the two points in each pair against each other.

15. A system according to claim 14 further comprising any one or more of the features of claims 2 to 7.

**Patentansprüche**

1. Ein System zur Messung von Veränderungen im Gang eines Subjekts, das System bestehend aus einer Messvorrichtung, die so eingerichtet ist, dass sie Veränderungen in der vertikalen Position des Subjekts misst, während das Subjekt eine Reihe von Schritten ausführt, einer Verarbeitungsvorrichtung und einer Anzeigevorrichtung, wobei die Verarbeitungsvorrichtung so eingerichtet ist, dass sie eine Vielzahl von Punkten in einem ersten Schritt und eine Vielzahl von Punkten in einem zweiten Schritt ermittelt, um eine Vielzahl von Punktepaaren zu ermitteln, jedes Paar bestehend aus einem Punkt für jeden der Schritte, um einen Höhenwert für jeden der Punkte in jedem der Paare zu bestimmen und die Anzeigevorrichtung zu steuern und eine Anzeige zu erzeugen, in der die Höhen der zwei Punkte in jedem Paar gegeneinander grafisch dargestellt werden.

2. Ein System gemäß Anspruch 1, wobei die Verarbeitungsvorrichtung so eingerichtet ist, dass sie den ersten und zweiten Schritt als aufeinanderfolgende Schritte in einer Reihe identifiziert.

3. Ein System gemäß Anspruch 1 oder Anspruch 2, wobei die Verarbeitungsvorrichtung so eingerichtet ist, dass sie jedes Punktepaar so identifiziert, dass das Zeitintervall zwischen den zwei Punkten in jedem Paar gleich ist.

4. Ein System gemäß eines der vorhergehenden Ansprüche, wobei die Verarbeitungsvorrichtung so eingerichtet ist, dass sie eine Reihe von Punkten durch die Reihe der Schritte identifiziert und dass jede Reihe von Punkten in eines der Paare eingeschlossen wird.

5. Ein System gemäß eines der vorhergehenden Ansprüche, wobei die Verarbeitungsvorrichtung so eingerichtet ist, dass sie ein Koordinatensystem definiert, mit zwei Achsen, die die Höhen der beiden Punkte in einem Paar darstellen, so dass die Höhen jedes Punktepaares durch eine Position innerhalb des Koordinatensystems dargestellt werden können, und die Verarbeitungsvorrichtung so eingerichtet ist, dass sie die Positionen im Koordinatensystem für jedes Punktepaar ermittelt und die Anzeige steuert, um diese Positionen anzuzeigen.

6. Ein System gemäß Anspruch 5, wobei die Verarbeitungsvorrichtung so eingerichtet ist, dass sie die mit den Punktepaaren verbundenen Positionen analysiert und einen Parameter der Positionen berechnet.

7. Ein System gemäß Anspruch 5 oder Anspruch 6, wobei die Verarbeitungsvorrichtung so eingerichtet ist, dass sie die Position einer Linie in einem vorbestimmten Verhältnis zu den mit den Punktepaaren verbundenen Positionen berechnet, und die Anzeige zur Darstellung der Linie steuert.

8. Ein Verfahren zur Messung von Veränderungen im Gang eines Subjekts, das Verfahren schließt dabei die Messung von Veränderungen in der vertikalen Position des Subjekts ein, während das Subjekt eine Reihe von Schritten ausführt, zur Ermittlung einer Vielzahl von Punkten in einem ersten Schritt und einer Vielzahl von Punkten in einem zweiten Schritt, zur Ermittlung einer Vielzahl von Punktepaaren, jedes Paar bestehend aus einem Punkt für jeden Schritt, Bestimmung eines Höhenwertes für jeden der Punkte in jedem der Paare, und Erzeugung einer Anzeige, die die Höhen der beiden Punkte in jedem Paar gegeneinander grafisch darstellt.

9. Ein Verfahren gemäß Anspruch 8, wobei der erste und der zweite Schritt als aufeinanderfolgende Schritte in der Reihe identifiziert werden.

10. Ein Verfahren gemäß Anspruch 8 oder Anspruch 9, wobei die Punktepaare ermittelt werden, so dass das Zeitintervall zwischen den beiden Punkten in jedem Paar gleich ist.

11. Ein Verfahren gemäß eines der Ansprüche 8 bis 10, einschließlich der Identifizierung einer Reihe von Punkten durch die Reihe von Schritten und einschließlich der jeweiligen Reihe von Punkten in einem der Paare.

12. Ein Verfahren gemäß eines der Ansprüche 8 bis 11, darüberhinaus bestehend aus der Definition eines Koordinatensystems mit zwei Achsen, die die Höhen von zwei Punkten in einem Paar repräsentieren, so dass die Höhen jedes Punktepaares durch eine Position innerhalb des Koordinatensystems repräsentiert werden kann, und der Identifizierung der Positionen im Koordinatensystem für jedes der Punktepaare, und Steuerung der Anzeige, um diese Positionen darzustellen.

13. Ein Verfahren gemäß Anspruch 11 oder Anspruch 12, bestehend aus der Analyse der mit den Punktepaaren verbundenen Positionen und der Berechnung eines Parameters der Positionen oder dem Einschluss der Berechnung der Position einer Linie mit einem vorbestimmten Verhältnis zu den mit den Punktepaaren verbundenen Positionen und Steuerung der Anzeige, um die Linie darzustellen.

14. Ein System zur Messung von Veränderungen im Gang eines Subjekts, das System bestehend aus einem Datenspeicher zum Speichern von Datenaufzeichnungsveränderungen in der vertikalen Position des Subjekts, während das Subjekt eine Reihe von Schritten ausführt, aus einer Verarbeitungsvorrichtung und Anzeigevorrichtung, wobei die Verarbeitungsvorrichtung so eingerichtet ist, dass sie eine Vielzahl von Punkten in einem ersten Schritt und eine Vielzahl von Punkten in einem zweiten Schritt identifiziert, um eine Vielzahl von Punktepaaren zu ermitteln, jedes Paar bestehend aus einem Punkt in jedem der Schritte, um einen Höhenwert für jeden der Punkte in jedem der Paare zu bestimmen, und um die Anzeige zu steuern und eine Anzeige zu erzeugen, die die Höhen der beiden Punkte in jedem Paar gegeneinander grafisch darstellt.

15. Ein System gemäß Anspruch 14, darüberhinaus bestehend aus einer oder mehreren der Eigenschaften in Anspruch 2 bis 7.

**Revendications**

1. Un système de mesure d'une variation dans la démarche d'un sujet, le système comprenant un moyen de mesure agencé de façon à mesurer des variations dans une position verticale du sujet pendant que le sujet effectue une série de pas, un moyen de traitement et un moyen d'affichage, où le moyen de traitement est agencé de façon à identifier une pluralité de points dans un premier des pas et une pluralité de points dans un deuxième des pas, de façon à identifier une pluralité de paires des points, chaque paire comprenant un point dans chacun des pas, de façon à déterminer une valeur de hauteur pour chacun des points dans chacune des paires, et de façon à commander le moyen d'affichage de façon à produire un affichage traçant les hauteurs des deux points dans chaque paire l'un par rapport à l'autre.

2. Un système selon la Revendication 1 où le moyen de traitement est agencé de façon à identifier les premier et deuxième pas en tant que pas consécutifs dans la série.

3. Un système selon la Revendication 1 ou 2 où le moyen de traitement est agencé de façon à identifier chaque paire de points de sorte que l'intervalle temporel entre les deux points dans chaque paire soit le même.

4. Un système selon l'une quelconque des Revendications précédentes où le moyen de traitement est agencé de façon à identifier une série de points dans la série de pas et à inclure chaque point de la série de points dans une des paires.

5. Un système selon l'une quelconque des Revendications précédentes où le moyen de traitement est agencé de façon à définir un système de coordonnées possédant deux axes représentant les hauteurs des deux points dans une paire, de sorte que les hauteurs de chaque paire de points puissent être représentées par une position à l'intérieur du système de coordonnées, et le moyen de traitement est agencé de façon à identifier les positions dans le système de coordonnées pour chacune des paires de points et à commander l'affichage de façon à indiquer ces positions.

6. Un système selon la Revendication 5 où le moyen de traitement est agencé de façon à analyser les positions associées aux paires de points et à calculer un paramètre des positions.

7. Un système selon la Revendication 5 ou 6 où le moyen de traitement est agencé de façon à calculer la position d'une ligne possédant une relation prédéterminée avec les positions associées aux paires des points et à commander l'affichage de façon à afficher la ligne.

8. Un procédé de mesure d'une variation dans la démarche d'un sujet, le procédé comprenant la mesure de variations dans une position verticale du sujet pendant que le sujet effectue une série de pas, l'identification d'une pluralité de points dans un premier des pas et d'une pluralité de points dans un deuxième des pas, l'identification d'une pluralité de paires des points, chaque paire comprenant un point dans chacun des pas, la détermination d'une valeur de hauteur pour chacun des points dans chacune des paires, et la production d'un affichage traçant les hauteurs des deux points dans chaque paire l'un par rapport à l'autre.

9. Un procédé selon la Revendication 8 où les premier et deuxième pas sont identifiés en tant que pas consécutifs dans la série.

10. Un procédé selon la Revendication 8 ou 9 où les paires de points sont identifiées de sorte que l'intervalle temporel entre les deux points dans chaque paire soit le même.

11. Un procédé selon l'une quelconque des Revendications 8 à 10 comprenant l'identification d'une série de points dans la série de pas et l'inclusion de chaque point de la série de points dans une des paires.

**12.** Un procédé selon l'une quelconque des Revendications 8 à 11 comprenant en outre la définition d'un système de coordonnées possédant deux axes représentant les hauteurs des deux points dans une paire, de sorte que les hauteurs de chaque paire de points puissent être représentées par une position à l'intérieur du système de coordonnées, l'identification des positions dans le système de coordonnées pour chacune des paires de points et la commande de l'affichage de façon à indiquer ces positions.

**13.** Un procédé selon la Revendication 11 ou 12 comprenant l'analyse des positions associées aux paires de points et le calcul d'un paramètre des positions, ou comprenant le calcul de la position d'une ligne possédant une relation prédéterminée avec les positions associées aux paires de points, et la commande de l'affichage de façon à afficher la ligne.

**14.** Un système de mesure d'une variation dans la démarche d'un sujet, le système comprenant une mémoire agencée de façon à conserver en mémoire des données enregistrant des variations dans une position verticale du sujet pendant que le sujet effectue une série de pas, un moyen de traitement et un moyen d'affichage, où le moyen de traitement est agencé de façon à identifier une pluralité de points dans un premier des pas et une pluralité de points dans un deuxième des pas, de façon à identifier une pluralité de paires des points, chaque paire comprenant un point dans chacun des pas, de façon à déterminer une valeur de hauteur pour chacun des points dans chacune des paires et de façon à commander le moyen d'affichage de façon à produire un affichage traçant les hauteurs des deux points dans chaque paire l'un par rapport à l'autre.

**15.** Un système selon la Revendication 14 comprenant en outre une quelconque ou plusieurs des caractéristiques selon les Revendications 2 à 7.

Fig. 1

14

10

12

Fig. 2

26

20

22

24

<u>Fig. 3</u>

<u>Fig. 4</u>

<u>Fig. 5</u>

Fig. 6

EP 2 713 874 B1

Fig. 7

Fig. 8

Fig. 9

## Fig. 10

a

b

c

d

## Fig. 11a

## Fig. 11b

<u>Fig. 12</u>

CoM Excursion $_{i-1}$ (cm)

CoM Excursion $_i$ (cm)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010073044 A